# EUROPEAN PATENT APPLICATION

(11) **EP 4 759 239 A1**
(43) Date of publication of application: **17.06.2026**
(21) Application number: 24851570.2
(22) Date of filing: 19.07.2024
(51) Int. Cl.: A61B 6/00, A61B 6/03, A61B 6/50

(54) **PROGRAM, INFORMATION PROCESSING METHOD, AND INFORMATION PROCESSING DEVICE**

(30) Priority: 09.08.2023 JP 2023130401
(71) Applicant: PRECISION IMAGING, INC., Tokyo 103-0025 (JP)
(72) Inventor: ISHII, Seiya, Tokyo 103-0025 (JP)
(74) Representative: Cabinet Beaumont
(86) International application number: PCT/JP2024/026001
(87) International publication number: WO 2025/033141

(57) **Abstract**

A program causes a computer to execute processing of: acquiring a fluoroscopic image of a patient undergoing a procedure related to an artificial hip joint; recognizing an inner plate line in the pelvis of the patient in the acquired fluoroscopic image, the inner plate line indicating a maximum reaming point when reaming the pelvis to place a cup included in the artificial hip joint; recognizing a target-to-reach line in the pelvis of the patient, the target-to-reach line indicating a minimum reaming point when reaming the pelvis to place the cup; deriving a cup CE angle when the cup is assumed to be placed with the pelvis of the patient reamed by a bone reamer used in the procedure related to the artificial hip joint; displaying the recognized inner plate line and the recognized target-to-reach line so as to be superimposed on the fluoroscopic image; and displaying the derived cup CE angle so as to be added to the fluoroscopic image.

## Description

### Technical Field

The present disclosure relates to a program, an information processing method, and an information processing apparatus.

This application claims priority under Japanese Patent Application No. 2023-130401 filed on August 9, 2023, and the entire content described in the Japanese application is incorporated herein.

### Background Art

An acetabular socket angle setting device for total hip arthroplasty is known (for example, Patent Literature 1). According to the acetabular socket angle setting device for total hip arthroplasty described in Patent Literature 1, a method for setting the acetabular socket angle using the pelvic surface and the inter-tear line as references is provided.

### Citation List

### Patent Literature:

Patent Literature 1: Japanese Patent Application Laid-Open Publication No. 2022-031047

### Summary

### Technical Problems

However, in the acetabular socket angle setting device for total hip arthroplasty described in Literature 1, no consideration is given to displaying an inner plate line, which indicates the maximum reaming point when reaming the pelvis to place the cup included in the artificial hip joint, so as to be superimposed on a fluoroscopic image of a patient undergoing a procedure related to the artificial hip joint.

The present disclosure has been made in view of the circumstances, and its purpose is to provide a program and the like capable of displaying an inner plate line, which indicates the maximum reaming point when reaming the pelvis to place the cup included in the artificial hip joint, so as to be superimposed on a fluoroscopic image of a patient undergoing a procedure related to the artificial hip joint.

### Solution to Problems

In one proposal, a program causes a computer to execute processing of: acquiring a fluoroscopic image of a patient undergoing a procedure related to an artificial hip joint; recognizing an inner plate line in a pelvis of the patient in the acquired fluoroscopic image, the inner plate line indicating a maximum reaming point when reaming the pelvis to place a cup included in the artificial hip joint; recognizing a target-to-reach line in the pelvis of the patient, the target-to-reach line indicating a minimum reaming point when reaming the pelvis to place the cup; deriving a cup CE angle when the cup is assumed to be placed with the pelvis of the patient reamed by a bone reamer used in the procedure related to the artificial hip joint; displaying the recognized inner plate line and the recognized target-to-reach line so as to be superimposed on the fluoroscopic image; and displaying the derived cup CE angle so as to be added to the fluoroscopic image.

In one proposal, an information processing method causes a computer to execute processing of: acquiring a fluoroscopic image of a patient undergoing a procedure related to an artificial hip joint; recognizing an inner plate line in a pelvis of the patient in the acquired fluoroscopic image, the inner plate line indicating a maximum reaming point when reaming the pelvis to place a cup included in the artificial hip joint; recognizing a target-to-reach line in the pelvis of the patient, the target-to-reach line indicating a minimum reaming point when reaming the pelvis to place the cup; deriving a cup CE angle when the cup is assumed to be placed with the pelvis of the patient reamed by a bone reamer used in the procedure related to the artificial hip joint; displaying the recognized inner plate line and the recognized target-to-reach line so as to be superimposed on the fluoroscopic image; and displaying the derived cup CE angle so as to be added to the fluoroscopic image.

In one proposal, an information processing apparatus includes: an acquisition unit that acquires a fluoroscopic image of a patient undergoing a procedure related to an artificial hip joint; an inner plate line recognition unit that recognizes an inner plate line in a pelvis of the patient in the acquired fluoroscopic image, the inner plate line indicating a maximum reaming point when reaming the pelvis to place a cup included in the artificial hip joint; a target-to-reach line recognition unit that recognizes a target-to-reach line in the pelvis of the patient, the target-to-reach line indicating a minimum reaming point when reaming the pelvis to place the cup; a derivation unit that derives a cup CE angle when the cup is assumed to be placed with the pelvis of the patient reamed by a bone reamer used in the procedure related to the artificial hip joint; and a display unit that displays the recognized inner plate line and the recognized target-to-reach line so as to be superimposed on the fluoroscopic image and displays the derived cup CE angle so as to be added to the fluoroscopic image.

### Advantageous Effects of Invention

According to the present disclosure, it is possible to display the inner plate line, which indicates the maximum reaming point when reaming the pelvis to place the cup included in the artificial hip joint, so as to be superimposed on the fluoroscopic image of the patient undergoing the procedure related to the artificial hip joint.

### Brief Description of Drawings

FIG. 1 is a schematic diagram showing an overview of an intraoperative support system including an information processing apparatus according to Embodiment 1.
FIG. 2 is a block diagram showing an example of the configuration of an information processing apparatus.
FIG. 3 is an explanatory diagram showing an example of a learning model (inner plate line model).
FIG. 4 is a flowchart showing an example of a processing procedure (during model training) of a processing unit of an information processing apparatus.
FIG. 5 is a flowchart showing an example of a processing procedure (during model operation) of a processing unit of an information processing apparatus.
FIG. 6 is an explanatory diagram of a cup CE angle.
FIG. 7 is an explanatory diagram of an inner plate line.
FIG. 8 is an explanatory diagram of a target-to-reach line.
FIG. 9 is an explanatory diagram of an upward limit line.
FIG. 10 is an explanatory diagram of a reach region (strike zone).
FIG. 11 is an explanatory diagram of the anteversion angle and abduction angle of a cup (which is assumed to placed with the pelvis reamed by a bone reamer).
FIG. 12 is an explanatory diagram illustrating a display screen of support information (support information superimposed screen).
FIG. 13 is an explanatory diagram illustrating a display screen for support information (distortion correction screen).

### Description of Embodiments

Hereinafter, the present invention will be described in detail with reference to the diagrams showing embodiments thereof.

### (Embodiment 1)

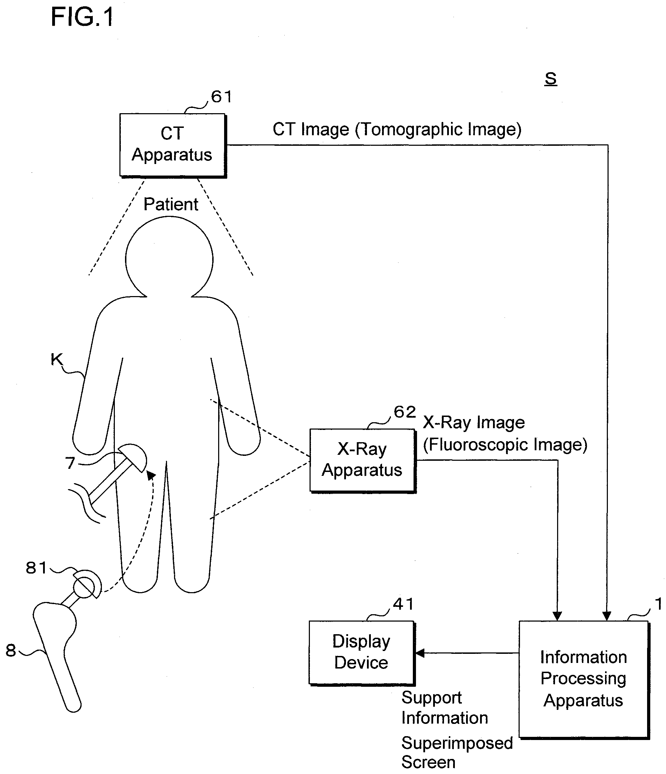

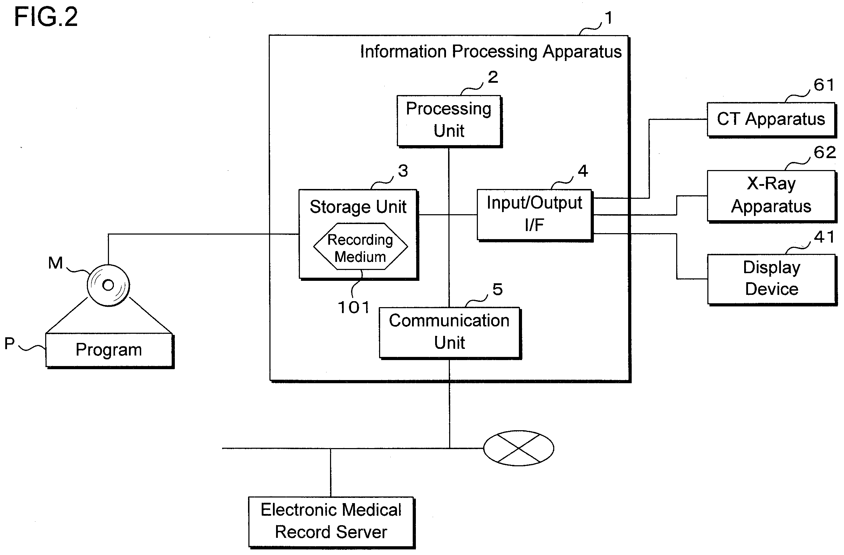

FIG. 1 is a schematic diagram showing an overview of an intraoperative support system S including an information processing apparatus 1 according to Embodiment 1. FIG. 2 is a block diagram showing an example of the configuration of the information processing apparatus 1. The intraoperative support system S is configured with the information processing apparatus 1 as its main apparatus, and a fluoroscopic image capturing apparatus such as an X-ray apparatus 62 and a tomographic image capturing apparatus such as a CT apparatus 61 are communicatively connected to the information processing apparatus 1. The information processing apparatus 1 acquires an X-ray image (fluoroscopic image) captured by the X-ray apparatus 62 in real time. The information processing apparatus 1 acquires a CT image (tomographic image) captured by the CT apparatus 61. In addition, the information processing apparatus 1 may be communicably connected to an electronic medical record server that stores and manages various kinds of medical data related to a patient K.

A CT image (tomographic image) of the patient K, who is to undergo a procedure related to an artificial hip joint 8, is captured by the CT apparatus 61 before the reaming of the pelvis using a bone reamer 7 is performed (preoperatively). An X-ray image (fluoroscopic image) of the patient K undergoing a procedure related to the artificial hip joint 8 is captured by the X-ray apparatus 62 during the reaming of the pelvis using the bone reamer 7 (during surgery). Therefore, the X-ray image includes the bone reamer 7 that is reaming the pelvis (during the reaming process).

After the doctor performs appropriate pelvic bone reaming with the bone reamer 7, the artificial hip joint 8, which includes a cup 81, is inserted (implanted) into the patient K's body. The artificial hip joint 8 includes the hemispherical cup 81 that is fitted into the reamed pelvis and a stem that is inserted into the femur. The bone reamer 7 has a hemispherical tip portion that performs reaming, and the outer edge shape of the tip portion is identical in shape and size to the outer edge shape of the cup 81 of the artificial hip joint 8.

As will be described in detail later, a processing unit 2 of the information processing apparatus 1 recognizes the shape of the bone reamer 7 (hemispherical tip portion that performs reaming) included in the acquired X-ray image using, for example, an object detection model such as YOLO or edge detection, and identifies the position, inclination, and the like of the bone reamer 7 in the pelvis. By regarding the identified position and inclination of the bone reamer 7 as the position and inclination of the cup 81 that is assumed to be placed with the pelvis reamed by the bone reamer 7, the processing unit 2 of the information processing apparatus 1 calculates and outputs various kinds of information regarding the cup 81 for which the placement is assumed. In addition, the processing unit 2 of the information processing apparatus 1 displays an inner plate line (reach region), which is for supporting the reaming by the bone reamer 7, so as to be superimposed on the X-ray image by using inner plate corresponding points (landmarks) derived from a plurality of preoperatively captured CT images using a learning model 101 (inner plate line model).

The information processing apparatus 1 is a computer capable of performing various kinds of information processing and information transmission/reception, and is, for example, a server apparatus or a personal computer. The server apparatus includes not only a single server apparatus but also a cloud server apparatus configured by a plurality of computers or a virtual server apparatus. When the information processing apparatus 1 is configured as, for example, a cloud server apparatus, the information processing apparatus 1 does not need to be installed in a medical facility where the patient K is located, like medical equipment such as the CT apparatus 61 or the X-ray apparatus 62, but may be connected to these medical equipment so as to be able to communicate with these through an external network such as the Internet. The information processing apparatus 1 includes the processing unit 2, a storage unit 3, an input/output I/F 4, and a communication unit 5.

The processing unit 2 includes one or more arithmetic processing devices with a timing function, such as a CPU (Central Processing Unit), an MPU (Micro-Processing Unit), and GPU (Graphics Processing Unit), and performs various kinds of information processing, control processing, and the like related to the information processing apparatus 1 by reading and executing a program P (program product) stored in the storage unit 3.

The storage unit 3 includes a volatile storage area, such as an SRAM (Static Random Access Memory), a DRAM (Dynamic Random Access Memory), and flash memory, and a non-volatile storage area, such as an EEPROM or a hard disk. The storage unit 3 stores in advance the program P (program product) and data to be referenced during processing. The program P (program product) stored in the storage unit 3 may be a program P (program product) read from a recording medium M that can be read by the information processing apparatus 1. In addition, the program P (program product) may be downloaded from an external computer (not shown) connected to a communication network (not shown) and stored in the storage unit 3.

The storage unit 3 stores various kinds of medical data related to the patient K and various values determined in the preoperative plan. The storage unit 3 further stores an actual file that forms the learning model 101 (inner plate line model). The actual file may also be configured as a part of the program P (program product).

The communication unit 5 is a communication module or communication interface for communicating, threshold wired or wireless means, with an electronic medical record server or an information terminal such as a smartphone held by medical personnel, and is, for example, a wired communication module such as an Ethernet (registered trademark) connector, a short-range wireless communication module such as Wi-Fi (registered trademark) or Bluetooth (registered trademark), or a wide-area wireless communication module such as 4G or 5G. The processing unit 2 communicates with an electronic medical record server or an information terminal through the communication unit 5, for example, through a local network within the medical institution or an external network such as the Internet.

The input/output I/F 4 is a communication interface that conforms to communication standards such as RS232C or USB. An input device such as a keyboard or a display device 41 such as a liquid crystal display is connected to the input/output I/F 4. In addition, medical equipment such as the CT apparatus 61 or the X-ray apparatus 62 may be connected to the input/output I/F 4.

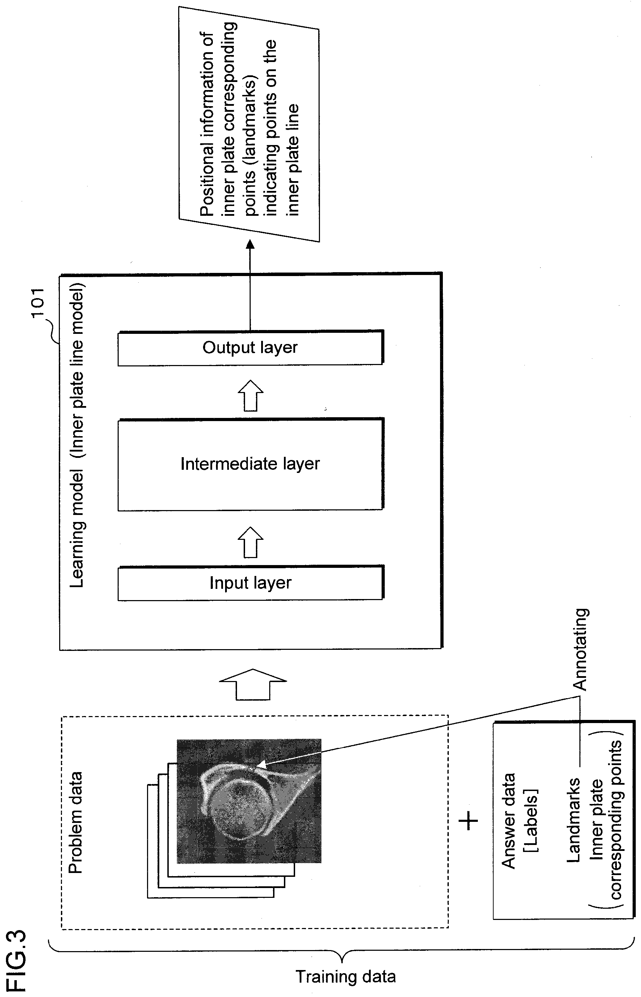

FIG. 3 is an explanatory diagram showing an example of the learning model 101 (inner plate line model). The learning model 101 (inner plate line model) is a neural network (NN) that is configured using, for example, RCNN (Regions with Convolutional Neural Network), Fast RCNN, Faster RCNN, SSD (Single Shot Multibook Detector), and YOLO (You Only Look Once) and performs object detection, semantic segmentation, or instance segmentation.

Based on the input image (tomographic image such as a CT image), the learning model 101 (inner plate line model) determines whether or not an inner plate corresponding point is included (yes/no), and outputs a region (positional information) of the inner plate corresponding point in the input image when an inner plate corresponding point is included (yes). That is, the learning model 101 functions as a region extraction model that extracts the region of the inner plate corresponding point included in the input image (tomographic image such as a CT image).

When the learning model 101 (inner plate line model) is configured as a neural network including a CNN (Convolutional Neural Network) that extracts image feature quantities, such as an RCNN, the input layer included in the learning model 101 (inner plate line model) has a plurality of neurons that receive the pixel values of the image and passes the input pixel values to the intermediate layer. The intermediate layer has a plurality of neurons that extract image feature quantities from an image, and passes the extracted image feature quantities to the output layer. The output layer has one or more neurons that output region information including the position of the inner plate corresponding point, and outputs the position (region coordinates or pixel number) of the inner plate corresponding point based on the image feature quantities output from the intermediate layer.

The neural network (learning model 101) trained using training data is intended to be used as a program module that is a part of artificial intelligence software. The learning model 101 is used in the information processing apparatus 1 including the processing unit 2 (CPU and the like) and the storage unit 3 as described above. When the learning model 101 is executed by the information processing apparatus 1 having such arithmetic processing capabilities, a neural network system is formed. That is, the processing unit 2 of the information processing apparatus 1 performs a calculation to extract the image feature quantities input to the input layer in accordance with an instruction from the learning model 101 stored in the storage unit 3, and outputs the position (region) of the inner plate corresponding point from the output layer.

The learning model 101 (inner plate line model) can be generated by preparing training data in which tomographic images (problem data) such as CT images including the pelvis (hip joint) are associated with labels (answer data) indicating the positions (regions) of inner plate corresponding points, which are points on the inner plate line in the pelvis, and performing machine learning on the untrained neural network using the training data. The training data is stored, for example, in the storage unit 3 of the information processing apparatus 1, and can be generated by collecting images and doctors' findings included in a large amount of diagnostic results or surgical results stored in electronic medical record servers at medical institutions such as hospitals. That is, the inner plate line (inner plate corresponding point, which is a point on the inner plate line) in the pelvis is a location (internal body part) identified based on the findings of doctors and others. According to the learning model 101 (inner plate line model) trained and configured in this manner, information indicating the position (region coordinates in the image coordinate system) of the inner plate corresponding point included in the tomographic image can be obtained by inputting a tomographic image such as a CT image to the learning model 101.

In addition, in the present embodiment, the learning model 101 (inner plate line model) has been described as being an RCNN, but the learning model 101 is not limited to the RCNN, and may be a learning model 101 constructed using other learning algorithms, such as a neural network other than the RCNN, an SVM (Support Vector Machine), a Transformer, YOLO, a Bayesian network, and a regression tree. The dataset of problem data and answer data included in the training data for training the learning model 101 is synonymous with the dataset of input data and output data when using the learning model 101, and if defined in either one of the datasets, this is undoubtedly applied to other datasets as well.

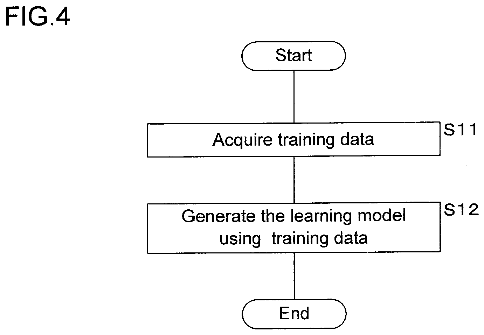

FIG. 4 is a flowchart showing an example of the processing procedure (during model training) of the processing unit 2 of the information processing apparatus 1. The processing unit 2 of the information processing apparatus 1 receives an operator's operation through, for example, a keyboard connected to the input/output I/F 4, and performs the following processing based on the received operation.

The processing unit 2 of the information processing apparatus 1 acquires training data (S11). The processing unit 2 of the information processing apparatus 1 acquires training data in which the positional information of inner plate corresponding points (landmarks) indicating points on the inner plate line is added to a tomographic image (CT image or the like). The positional information of the inner plate corresponding points (landmarks) may be obtained, for example, by annotating the tomographic image with a figure, such as a circle, at a location (a part of the pelvis) indicating the inner plate corresponding point.

The processing unit 2 of the information processing apparatus 1 generates the learning model 101 (inner plate line model) using the acquired training data (S12). The processing unit 2 of the information processing apparatus 1 generates the learning model 101 (inner plate line model) by inputting the training data (an image in which the location indicating the inner plate corresponding point is annotated on the tomographic image) to an untrained neural network to train the neural network.

In the present embodiment, the learning model 101 outputs the positional information of the inner plate corresponding points (landmarks) indicating points on the inner plate line based on the input CT image (displays the inner plate corresponding points (landmarks) so as to be superimposed on the CT image). However, the present disclosure is not limited thereto. Based on the input CT image, the learning model 101 may output, in addition to the inner plate corresponding points (landmarks) indicating points on the inner plate line, target-to-reach corresponding points indicating points on the target-to-reach line and upward limit points indicating points on the upward limit line, which will be described below. In this case, in addition to the CT image, a target CE angle may be input to the learning model 101. That is, the learning model 101 may function as a reach region model that outputs points on each of the inner plate line, the target-to-reach line, and the upward limit line (inner plate corresponding point, target-to-reach corresponding point, upward limit point) for identifying the reach region based on the input CT image (or the CT image and the target CE angle). In this case, the reach region model is trained using training data including answer data annotated with inner plate corresponding points, target-to-reach corresponding points, and upward limit points for the CT image that is problem data.

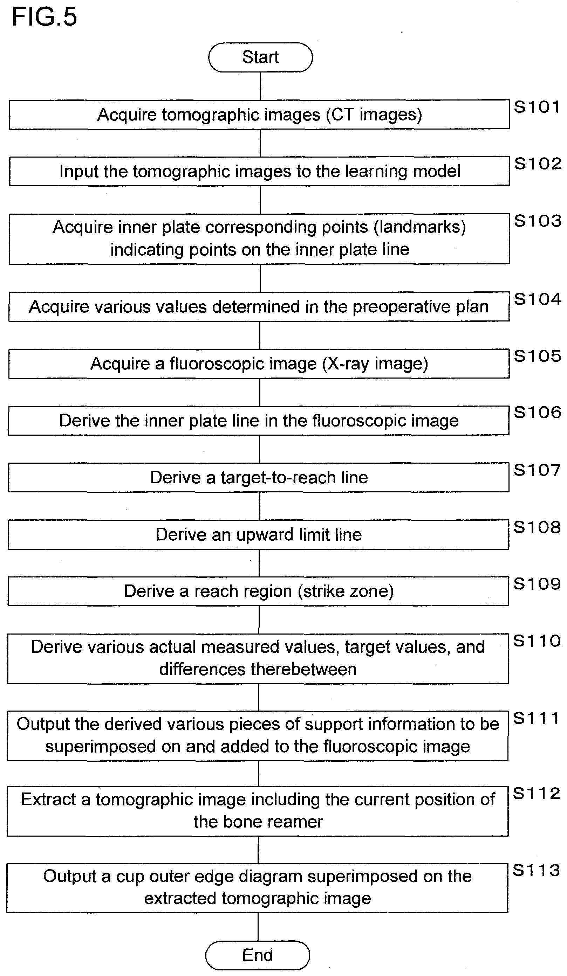

FIG. 5 is a flowchart showing an example of the processing procedure (during model operation) of the processing unit 2 of the information processing apparatus 1. The processing unit 2 of the information processing apparatus 1 starts processing of the flowchart, for example, when a fluoroscopic image is input as a trigger or when a start command is received from an input device such as a keyboard connected to the input/output I/F 4.

The processing unit 2 of the information processing apparatus 1 acquires tomographic images (CT images) obtained by imaging the pelvis of the patient K (S101). Before the reaming procedure using the bone reamer 7 (preoperatively), tomographic images (CT images or MRI images) of the internal body part including the pelvis and hip joint of the patient K are captured by using a tomographic imaging apparatus such as the CT apparatus 61 or an MRI apparatus. The tomographic images captured preoperatively are stored, for example, in the storage unit 3 of the information processing apparatus 1. Alternatively, these tomographic images (CT images and the like) may be stored in an electronic medical record server, and the processing unit 2 of the information processing apparatus 1 may access the electronic medical record server using, for example, a patient KID that uniquely identifies the patient K and acquire the tomographic images (CT images and the like) from the electronic medical record server. The tomographic images include a plurality of tomographic images sliced at a predetermined slice width (in the vertical (Y-axis) direction of the human body). That is, in the present embodiment, the Y-axis indicates the upper limit direction of the human body, and the positive direction on the Y-axis indicates upward. The X-axis indicates the left-right direction of the human body (positive direction is the right direction on the paper), and the Z-axis indicates the front-back direction of the human body (positive direction is forward).

The processing unit 2 of the information processing apparatus 1 inputs the tomographic images to the learning model 101 (inner plate line model) (S102). The processing unit 2 of the information processing apparatus 1 inputs each of the acquired plurality of tomographic images to the learning model 101 (inner plate line model). The learning model 101 (inner plate line model) is trained to output the positional information (landmarks) of the inner plate (inner plate corresponding points on the inner plate line) when a tomographic image is input. The learning model 101 (inner plate line model) outputs the positional information (landmarks) of the inner plate, based on the input tomographic image, so as to be superimposed on the input tomographic image.

The processing unit 2 of the information processing apparatus 1 acquires inner plate corresponding points (landmarks) indicating points on the inner plate line from the learning model 101 (S103). The processing unit 2 of the information processing apparatus 1 acquires, from the learning model 101, tomographic images on which inner plate corresponding points (landmarks) indicating points on the inner plate line are superimposed, and stores the acquired plurality of tomographic images (tomographic images on which inner plate corresponding points are displayed so as to be superimposed) in the storage unit 3.

The processing unit 2 of the information processing apparatus 1 acquires various values determined in the preoperative plan by referring to the storage unit 3 (S104). The various values determined in the preoperative plan include, for example, the radius of the cup 81 of the artificial hip joint 8 to be placed in the body, the target CE angle, the target distance between the outer edge of the cup 81 and the inner plate line set in advance as a target value, the target anteversion angle, and the target abduction angle.

The target CE angle indicates a cup CE angle when the cup 81 is placed, which is set in advance as a target value. The target distance indicates a distance between the cup 81 set in advance as a target value and the inner plate line derived using the learning model 101 (inner plate line model) based on the tomographic image. The target anteversion angle indicates the anteversion angle of the cup 81 when the cup 81 is placed, which is set in advance as a target value. The target abduction angle indicates the abduction angle of the cup 81 when the cup 81 is placed, which is set in advance as a target value. The various values determined in the preoperative plan are determined by doctors and others, input in advance to the information processing apparatus 1, and stored in the storage unit 3. Alternatively, the processing unit 2 of the information processing apparatus 1 may acquire various values determined in the preoperative plan for the patient K from an electronic medical record system based on the patient KID or the like.

The processing unit 2 of the information processing apparatus 1 acquires a fluoroscopic image (X-ray image), which is obtained by capturing the pelvis of the patient K, during surgery for the patient K undergoing a procedure related to the artificial hip joint 8 (S105). The processing unit 2 of the information processing apparatus 1 acquires, in real time, a fluoroscopic image (X-ray image) obtained by capturing the pelvis of the patient K during surgery for the patient K undergoing a procedure related to the artificial hip joint 8, that is, while the pelvis is being reamed by the bone reamer 7. The processing unit 2 of the information processing apparatus 1 may acquire a fluoroscopic image (X-ray image) in real time or may acquire a fluoroscopic image (X-ray image) in the form of a moving image. In this manner, the fluoroscopic image (X-ray image) obtained by capturing the patient K in real time during surgery includes the bone reamer 7 for reaming the pelvis. Therefore, it is possible to identify the current position and inclination of the bone reamer 7 (the outer edge of the hemispherical tip portion that performs reaming). When it is assumed that the cup 81 is placed at the current position of the bone reamer 7 in the pelvis, that is, in the cavity (installation space) of the bone reamed by the bone reamer 7, it is possible to derive the cup CE angle, anteversion angle, and abduction angle of the assumedly placed cup 81.

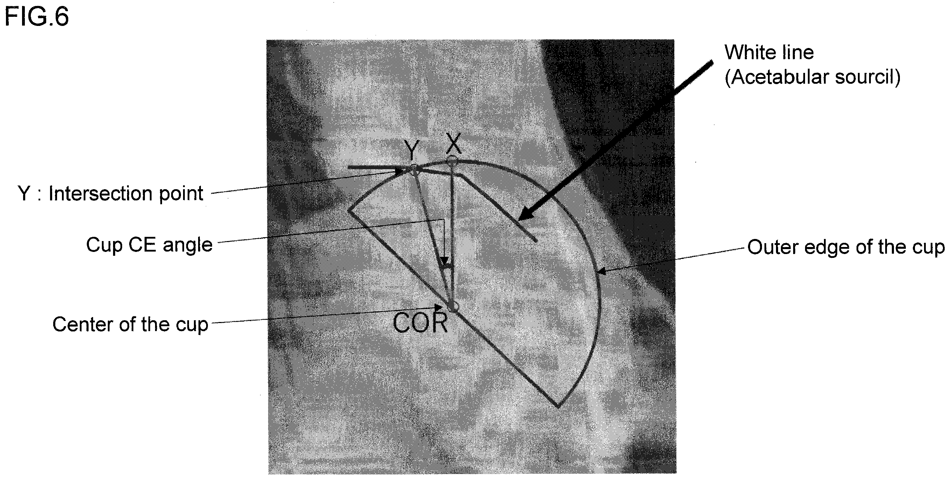

FIG. 6 is an explanatory diagram of the cup CE angle. In the illustration of the present embodiment, based on the current position of the bone reamer 7 in the pelvis, a semicircular figure object indicating the cup 81 assumed to be placed is displayed so as to be superimposed on the fluoroscopic image. The processing unit 2 of the information processing apparatus 1 derives, as the cup CE angle, an angle formed by a line, which connects the center of rotation (COR) of the cup 81 assumed to be placed with the pelvis reamed by the bone reamer 7 to the intersection point between the outer edge of the cup 81 and the white line (Acetabular sourcil) of the pelvic weight-bearing surface, and the vertical line (Y-axis) with respect to the pelvic reference line, which connects the lower ends of the left and right teardrops in the pelvis.

The processing unit 2 of the information processing apparatus 1 derives the inner plate line in the fluoroscopic image based on the inner plate corresponding points (landmarks) output from the learning model 101 (S106). The learning model 101 outputs a tomographic image obtained by superimposing the inner plate corresponding points (landmarks) on the input tomographic image (CT image or the like). The inner plate corresponding point is a maximum reaming point when reaming the pelvis, that is, a point located on the inner plate line indicating the reaming limit line that should not be exceeded. The processing unit 2 of the information processing apparatus 1 extracts each of the points (landmarks) shown in each of the tomographic images, and derives (identifies) the inner plate line by forming (connecting) a line connecting the extracted plurality of points (landmarks).

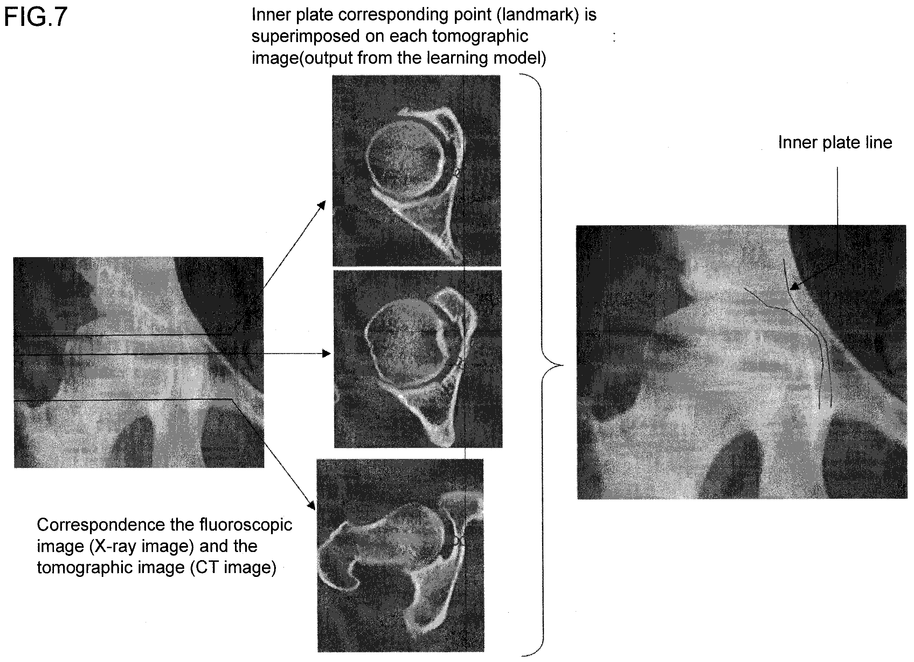

FIG. 7 is an explanatory diagram of the inner plate line. In the illustration of the present embodiment, a plurality of (three in this example) tomographic images are shown, and each tomographic image has a correspondence (the same Y coordinate) with a fluoroscopic image (X-ray image) at a plurality of locations in the Y-axis direction (up and down direction of the human body). Each of these tomographic images is output from the learning model 101 (inner plate line model), and a circle indicating an inner plate corresponding point (landmark) is superimposed on each tomographic image. The processing unit 2 of the information processing apparatus 1 identifies a plurality of inner plate corresponding points (landmarks) in the fluoroscopic image by using each of the tomographic images on which inner plate corresponding points (landmarks) are superimposed as described above, and forms an inner plate line by connecting the identified plurality of inner plate corresponding points.

The processing unit 2 of the information processing apparatus 1 may generate (reconstruct) a three-dimensional medical image including the pelvis and hip joint of the patient K by using volume data constructed using the plurality of tomographic images. On this basis, the processing unit 2 of the information processing apparatus 1 may set a three-dimensional internal body coordinate system in the tomographic image or three-dimensional medical image including the patient K's pelvis and the like. In the internal body coordinate system, the processing unit 2 of the information processing apparatus 1 may define, for example, a line connecting the lower ends of two teardrops of the pelvis as a pelvic reference line, define the horizontal direction (left-right direction of the human body) with respect to the pelvic reference line as the X-axis, define the vertical direction (up-down direction of the human body) with respect to the pelvic reference line as the Y-axis, and defines a direction perpendicular to the X-axis and the Y-axis (front-back direction of the human body) as the Z-axis, and perform various types of arithmetic processing. The processing unit 2 of the information processing apparatus 1 may perform normalization of the internal body coordinate system, that is, alignment, between the fluoroscopic image (X-ray image) and the tomographic image (CT image) by using the shape recognition results of the same internal body part (for example, teardrop) included in both the acquired fluoroscopic image (X-ray image) and the acquired tomographic image (CT image). As a result, the processing unit 2 of the information processing apparatus 1 applies the internal body coordinate system set in the plurality of tomographic images (or the reconstructed three-dimensional medical image) to the fluoroscopic image (X-ray image) captured in real time during surgery, and derives an inner plate line connecting a plurality of inner plate corresponding points in the fluoroscopic image (X-ray image) based on the inner plate corresponding points (landmarks) identified in each tomographic image. In addition, the processing unit 2 of the information processing apparatus 1 can also identify the current position of the bone reamer 7 included in the fluoroscopic image (X-ray image) in the internal body coordinate system, which is a coordinate system shared with the tomographic image (CT image), by performing conversion from the image coordinate system of the fluoroscopic image to the internal body coordinate system.

The processing unit 2 of the information processing apparatus 1 derives a target-to-reach line in the fluoroscopic image according to the radius of the cup 81 and the target CE angle (S107). The radius of the cup 81 of the artificial hip joint 8 to be inserted into the patient K and the target CE angle are stored in the storage unit 3 as various values determined in the preoperative plan. The processing unit 2 of the information processing apparatus 1 derives a target-to-reach line in the fluoroscopic image based on the white line (Acetabular sourcil) formed by the inner edge of the pelvic weight-bearing surface included in the fluoroscopic image, according to the radius of the cup 81 and the target CE angle. The white line (Acetabular sourcil) formed by the inner edge of the pelvic weight-bearing surface can be identified (its position can be grasped) by performing shape recognition processing on the fluoroscopic image. The target-to-reach line indicates the minimum reaming point when reaming the pelvis of the patient K to place the cup 81 in the pelvis.

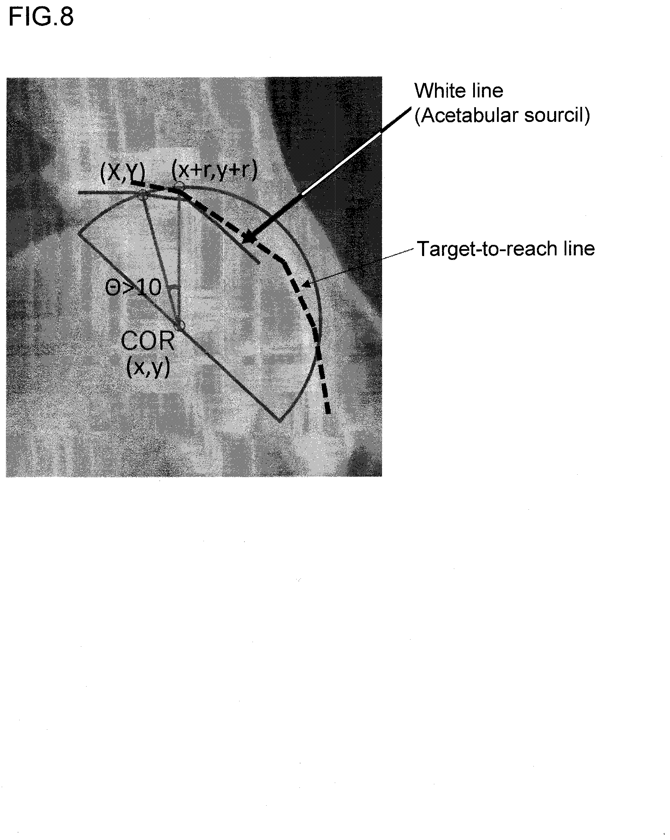

FIG. 8 is an explanatory diagram of the target-to-reach line. The storage unit 3 of the information processing apparatus 1 stores various values (parameters) that are determined in the preoperative plan when performing a procedure related to the artificial hip joint 8, such as a minimum CE angle (for example, 10°) set in advance to stabilize the cup 81 to be placed and the radius of the cup 81 to be implanted. The processing unit 2 of the information processing apparatus 1 acquires these various values determined in the preoperative plan by referring to the storage unit 3.

The processing unit 2 of the information processing apparatus 1 defines the intersection 1 (X, Y) between the center (x, y) of the cup 81, which is assumed to be placed in the cavity (installation space) of the bone reamed by the bone reamer 7, and the white line (Acetabular sourcil) formed by the inner edge of the pelvic weight-bearing surface when reamed. In this case, the center (x, y) of the cup 81 is expressed by the following equation using the intersection point (X, Y), the radius (r) of the cup 81, and the cup CE angle (Θ). The X coordinate (x) of the center of the cup 81 is calculated by subtracting a value, which is obtained by multiplying the radius (r) of the cup 81 by the sine of the cup CE angle (Θ), from the X coordinate (X) of the intersection point (x = X - r*sinΘ). The Y coordinate (y) of the center of the cup 81 is calculated by subtracting a value, which is obtained by multiplying the radius (r) of the cup 81 by the cosine of the cup CE angle (Θ), from the Y coordinate (Y) of the intersection point (y = Y - r*cosΘ). Thus, the center (x, y) of the cup 81 corresponds to the intersection point (X, Y) in a one-to-one manner. When the intersection point (X, Y) changes, that is, when the white line (Acetabular sourcil) formed by the inner edge of the pelvic weight-bearing surface during reaming changes according to the amount of reaming performed by the bone reamer 7, the center (x, y) of the cup 81 also changes. When changing the intersection point (X, Y) in this manner, satisfying the condition that the cup CE angle Θ exceeds the minimum CE angle (10°) (Θ > 10) is a requirement for implantation. Therefore, the processing unit 2 of the information processing apparatus 1 determines, within a range satisfying this requirement (Θ > 10), a variation range in which the intersection point (X, Y) can change.

The processing unit 2 of the information processing apparatus 1 identifies, using the above equations, a region where the center (x, y) of the cup 81 can be located, based on the determined variation range of the intersection point (X, Y). The processing unit 2 of the information processing apparatus 1 derives, as a line (target-to-reach line) that the outer edge of the bone reamer 7 (the outer edge of the hemispherical tip portion that performs reaming) should touch in order to satisfy the condition (Θ > 10), an edge line within the range of possible points (x', y') indicating each point on the outer edge of the cup 81 by taking into account the radius (r) of the cup 81 with respect to the center (x, y) of the cup 81 determined according to the changed intersection point (X, Y). In deriving the target-to-reach line, the processing unit 2 of the information processing apparatus 1 selects an arbitrary point (x1, y1) on the acetabular floor of the pelvis before reaming by the bone reamer 7, and defines the angle of the X-axis with respect to the line connecting the center (x, y) of the cup 81 and the arbitrary point as α. The coordinates of the arbitrary point are (x + rcosα, y + rsinα). On this basis, the processing unit 2 of the information processing apparatus 1 may derive a target-to-reach line so that the point (x + rcosα, y + rsinα) taking into account the radius (r) of the cup 81 for the center (x, y) of the cup 81 corresponding to the changed intersection point (X, Y) is greater than the coordinates (x1, y1) of the arbitrary point (x + rcosα > x1 and y + rsin α > y2). That is, the processing unit 2 of the information processing apparatus 1 derives, as the target-to-reach line, a range of (x', y') that the center (x, y) of the cup 81 satisfying the conditional expressions "(x + rcosα > x1 and y + rsinα > y2)" and "cup CE angle; Θ > 10" can take, that is, the edge of the range that can be taken as the outer edge of the cup 81 by taking into account the radius (r) of the cup 81. The target-to-reach line derived in this manner corresponds to the inner line in the reach region (the inner side on the near side in the reaming direction of the bone reamer 7).

The processing unit 2 of the information processing apparatus 1 derives an upward limit line in the fluoroscopic image according to the center of the femoral head on the healthy side (S108). The processing unit 2 of the information processing apparatus 1 recognizes the femoral head on the healthy side included in the fluoroscopic image, and derives an upward limit line in the fluoroscopic image according to the center of the femoral head on the healthy side. The upward limit line is a line indicating the limit of upward movement that the center of the femoral head on the affected side (the center of the head ball of the artificial hip joint 8) can take with respect to the center of the femoral head on the healthy side.

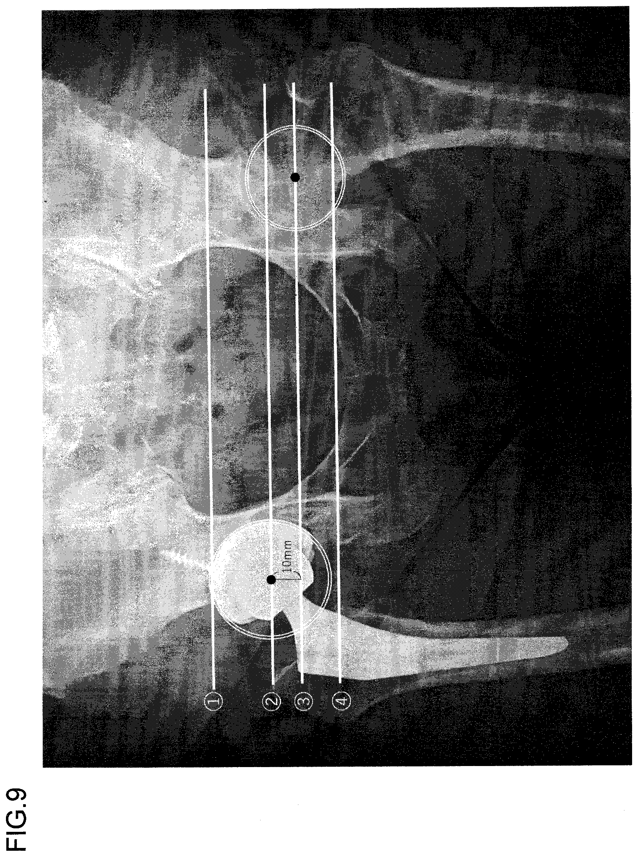

FIG. 9 is an explanatory diagram of the upward limit line. In the illustration of the present embodiment, four line diagrams ((1) to (4)) are shown for reference. These line diagrams are shown as follows. Line diagram (1) shows a "line parallel to the X-axis (cup-edge upward limit line) that passes through y' = y2 + 10 mm + cup radius mm". Line diagram (2) shows a "line parallel to the X-axis that passes through y = y2 + 10 mm (cup-center upward limit line) ". Line diagram (3) shows a "line parallel to the X-axis that passes through the healthy-side femoral head center (x2, y2)". Line diagram (4) shows a "pelvic reference line (X-axis) connecting the lower ends of the teardrops". For the patient K undergoing a procedure related to the artificial hip joint 8, it is generally assumed that the procedure related to the artificial hip joint 8 (implant) is performed on one of the left or right legs. In this case, the affected part side on which the procedure related to the artificial hip joint 8 is performed is referred to as an affected side, and the healthy part side on which the procedure related to the artificial hip joint 8 is not performed is referred to as a healthy side.

The processing unit 2 of the information processing apparatus 1 recognizes the shape of the femoral head on the healthy side included in the acquired fluoroscopic image (X-ray image) using, for example, an object detection model such as YOLO or edge detection, and derives the center of the femoral head on the healthy side based on the outer edge that forms an arc shape at the femoral head, for example, by calculating the center of curvature using a plurality of points located on the outer edge. The processing unit 2 of the information processing apparatus 1 derives the upward limit line (line segment (2)) for the cup center (the femoral head center of the implant on the affected side), which is within a predetermined value (for example, 10 mm) in the positive direction (upward) of the Y-axis, with respect to a line (line segment (3)) that passes through the derived femoral head center (x2, y2) on the healthy side and is parallel to the X-axis (line segment (4)). The upward limit line of the cup center (the femoral head center of the implant on the affected side) serves as a reference line for determining the cup-edge upward limit line (line segment (1)), that is, the upward limit line (line segment (1)) in the reach region (strike zone).

The femoral head center on the affected side, where a procedure related to the artificial hip joint 8 is performed, corresponds to the center (x, y) of the cup 81 to be placed. The Y-axis indicates the upper limit direction of the human body, and the positive direction of the Y-axis indicates upward. The Y coordinate (y) of the center (x, y) of the cup 81 to be placed is set within a range not exceeding, for example, 10 mm (y < y2 + 10[mm]) upward from the Y coordinate (y2) of the center of the femoral head on the healthy side. Therefore, the processing unit 2 of the information processing apparatus 1 derives an upward limit line (line segment (2)) of the cup center (the femoral head center of the implant on the affected side) that passes through a point moved upward (along the positive direction of the Y-axis) by the upward limit value (for example, 10 mm) from the Y coordinate (y2) of the femoral head center on the healthy side and is perpendicular to the Y coordinate. In addition, the cup-edge upward limit line (line segment (1)), which is parallel to the upward limit line (line segment (2)) of the cup center (the femoral head center of the implant on the affected side) (that is, parallel to the X-axis) and located upward (in the positive direction of the Y-axis) by the cup radius, that is, the upward limit line (line segment (1)) in the reach region (strike zone) is determined (derived). In this manner, by deriving the upward limit line (line segment (1): cup-edge upward limit line) in the reach region (strike zone) based on the femoral head center on the healthy side, it is possible to provide doctors and others with a guideline (upward limit line in pelvic reaming) for positioning the femoral head center on the affected side (the center of the femoral head ball of the artificial hip joint 8).

The processing unit 2 of the information processing apparatus 1 derives a reach region (strike zone) based on the derived inner plate line, target-to-reach line, and upward limit line (S109). Based on the derived upward limit line, inner plate line, and target-to-reach line, the processing unit 2 of the information processing apparatus 1 derives a region surrounded by these lines as a reach region (strike zone).

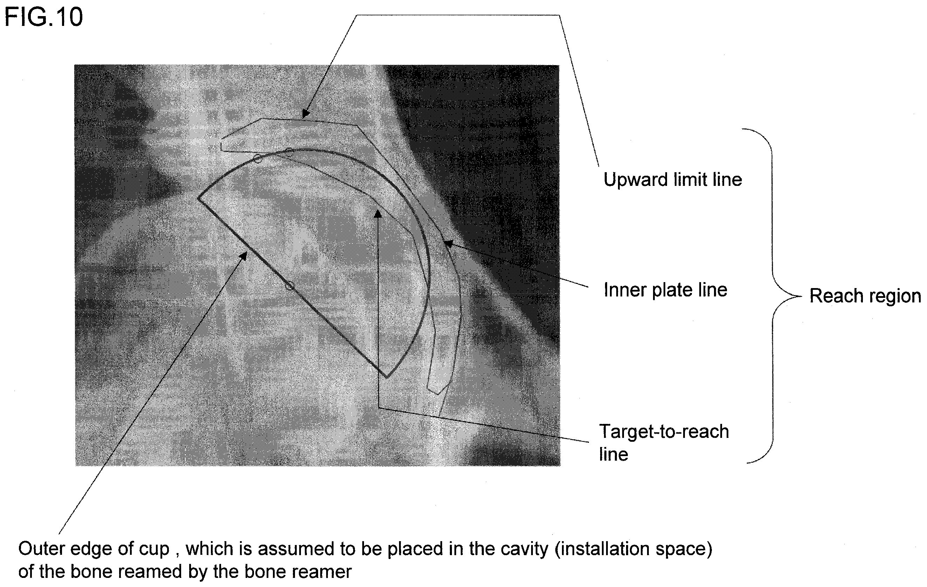

FIG. 10 is an explanatory diagram of the reach region (strike zone). The reach region indicates a region where stable fixation of the cup 81 is ensured if a part of the cup 81 is in contact with the reach region when the cup 81 is placed. In the reach region, the upward limit line and the inner plate line indicate limit lines that should not be exceeded during reaming by the bone reamer 7. By displaying the reach region derived as described above so as to be superimposed on the fluoroscopic image, the positional relationship between the current position of the bone reamer 7 and a region (reach region) that the bone reamer 7 should reach through reaming can be provided to the doctor in real time using the bone reamer 7 included in the fluoroscopic image displayed in real time.

The processing unit 2 of the information processing apparatus 1 derives various actual measured values, target values, and differences therebetween at the current time (S110). Using the fluoroscopic image, the processing unit 2 of the information processing apparatus 1 derives the actual measured values of the cup 81, which is assumed to be placed with the pelvis reamed by the bone reamer 7. The target CE angle and the target distance between the outer edge of the cup 81 and the inner plate line are set in advance as target values, and are stored in the storage unit 3 of the information processing apparatus 1 as various values determined in the preoperative plan when performing a procedure related to the artificial hip joint 8. The processing unit 2 of the information processing apparatus 1 acquire these various values determined in the preoperative plan by referring to the storage unit 3.

By using the acquired fluoroscopic image, the processing unit 2 of the information processing apparatus 1 identifies the position and inclination of the cup 81 when the cup 81 is assumed to be placed with the pelvis reamed by the bone reamer 7, based on the current position and inclination of the bone reamer 7 (the outer edge of the hemispherical tip portion that performs reaming) in the pelvis of patient K. Examples of the inclination include the anteversion angle and the abduction angle of the cup 81.

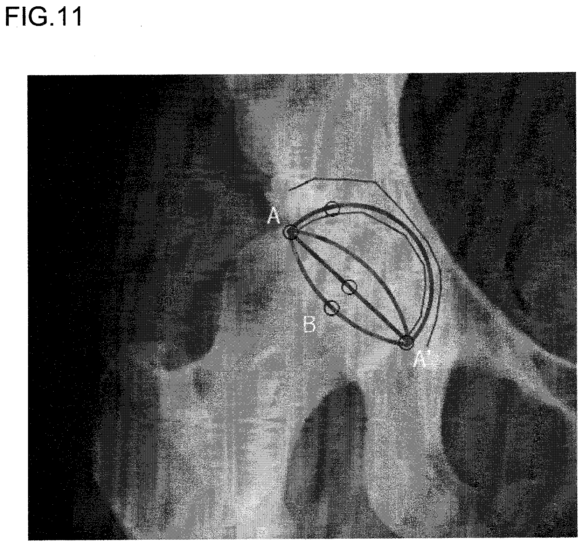

FIG. 11 is an explanatory diagram of the anteversion angle and the abduction angle of the cup 81 (assumed to be placed with the pelvis reamed by the bone reamer 7). The processing unit 2 of the information processing apparatus 1 identifies the three points (A, A', B) of the ellipse through shape recognition processing. In this case, in the fluoroscopic image, assuming that the angle between A-A' and A'-B is X, the anteversion angle is expressed as "sin^(-1)*tanX". The processing unit 2 may recognize the shape and current position of the bone reamer 7 (the outer edge of the hemispherical tip portion that performs reaming) corresponding to the cup 81 in the assumed placement by performing calculations using the image coordinate system in the fluoroscopic image, thereby calculating the anteversion angle and the abduction angle.

The processing unit 2 of the information processing apparatus 1 may derive the target anteversion angle and the target abduction angle based on the target CE angle set in advance. The processing unit 2 of the information processing apparatus 1 may derive the target anteversion angle and the target abduction angle by referring to a table (target CE angle table) in which the values of the target anteversion angle and the target abduction angle are associated with each value of the target CE angle. Various lookup tables that the processing unit 2 of the information processing apparatus 1 refers to when performing various arithmetic processing, including the target CE angle table, are stored in the storage unit 3.

The processing unit 2 of the information processing apparatus 1 outputs the derived various pieces of support information (reach region, difference between the actual measured value and the target value) so as to be superimposed on and added to the fluoroscopic image (S111). The support information includes, for example, a reach region and a difference between the actual measured value and the target value. The processing unit 2 of the information processing apparatus 1 outputs the derived reach region to the display device 41 in such a manner that the derived reach region is superimposed on the fluoroscopic image. The processing unit 2 of the information processing apparatus 1 further outputs various actual measured values, target values, and differences between the actual measured values and target values to the display device 41 in such a manner that these are added to the fluoroscopic image. When outputting this information, the processing unit 2 of the information processing apparatus 1 may generate and output screen data that configures a display screen (support information superimposed screen). The processing unit 2 of the information processing apparatus 1 displays the derived actual measured values (cup CE angle, actual measured distance between the outer edge of the cup 81 and the inner plate line, anteversion angle, abduction angle) and the corresponding target values (target CE angle, target distance between the outer edge of the cup 81 and the inner plate line, target anteversion angle, target abduction angle) so as to be added to the fluoroscopic image (for example, display using a sub-screen or a separate frame), in such a manner that these are compared with each other. As a result, doctors and others can efficiently grasp the differences between various actual measured values and target values at the current time.

The processing unit 2 of the information processing apparatus 1 extracts a tomographic image including the current position of the bone reamer 7 from among a plurality of tomographic images obtained by imaging the pelvis of the patient K (S112). The processing unit 2 of the information processing apparatus 1 identifies the current position of the bone reamer 7 (the outer edge of the hemispherical tip portion that performs reaming) in the pelvis of the patient K by performing shape recognition on the acquired fluoroscopic image using, for example, an object detection model such as YOLO or edge detection. The fluoroscopic image is aligned with a tomographic image (such as a CT image), so that the processing unit 2 of the information processing apparatus 1 can apply the internal body coordinate system defined in the tomographic image to the fluoroscopic image.

The processing unit 2 of the information processing apparatus 1 may acquire the current position of the bone reamer 7 (the outer edge of the hemispherical tip portion that performs reaming) included in the fluoroscopic image by identifying it in the internal body coordinate system defined in the tomographic image. The storage unit 3 of the information processing apparatus 1 stores a plurality of tomographic images (CT images and the like), which are obtained by capturing the patient K with the CT apparatus 61 before the pelvis is reamed by the bone reamer 7. The plurality of tomographic images (CT images and the like) are images sliced in the Y-axis direction, that is, in the front-back direction of the human body, and each of the tomographic images (CT images and the like) is represented by the X-axis (left-right direction of the human body) and the Z-axis (front-back direction of the human body).

The processing unit 2 of the information processing apparatus 1 extracts one or more tomographic images (CT images and the like) including the current position of the bone reamer 7 identified in the fluoroscopic image, according to the current position of the bone reamer 7. The processing unit 2 of the information processing apparatus 1 extracts a tomographic image (CT image and the like) corresponding to the Y coordinate (y) of the center of the bone reamer 7 (the outer edge of the hemispherical tip portion that performs reaming), that is, the center (x, y) of the cup 81 at the location where the cup 81 is assumed to be placed in the current reaming state. Alternatively, since each tomographic image is associated with a Y-axis coordinate corresponding to the tomographic image and a region (range) of the XZ plane, for example, using the internal body coordinate system, the processing unit 2 of the information processing apparatus 1 may extract a tomographic image corresponding to the current position of the bone reamer 7 in the internal body coordinate system, for example.

The processing unit 2 of the information processing apparatus 1 outputs a cup outer edge diagram when the cup 81 is assumed to be placed with the pelvis reamed by the bone reamer 7, in such a manner that the cup outer edge diagram is superimposed on the extracted tomographic image (S113). The processing unit 2 of the information processing apparatus 1 outputs a semicircular figure (cup outer edge diagram) indicating the current position of the bone reamer 7 (the outer edge of the hemispherical tip portion that performs reaming) in such a manner that the semicircular figure (cup outer edge diagram) is superimposed on one or more extracted tomographic images (CT images and the like). The processing unit 2 of the information processing apparatus 1 may further derive the installation angle and depth of the cup 81 at the location where the cup 81 is assumed to be installed in the current reaming state by using the fluoroscopic image, and further output the derived installation angle and depth so as to be superimposed on the tomographic image (CT image and the like). In this manner, the processing unit 2 of the information processing apparatus 1 extracts a corresponding tomographic image (CT image and the like) according to the current position of the bone reamer 7 identified in the X-ray image (the location where the cup 81 is assumed to be placed in the current reaming state), and outputs the tomographic image in such a manner that the outer edge diagram (semicircular figure object) of the cup 81 assumed to be placed and the installation angle and depth of the cup 81 are superimposed on the tomographic image. Therefore, it is possible to provide doctors and others with information to support three-dimensional intraoperative recognition, including information about the Z-axis (front-back direction of the human body).

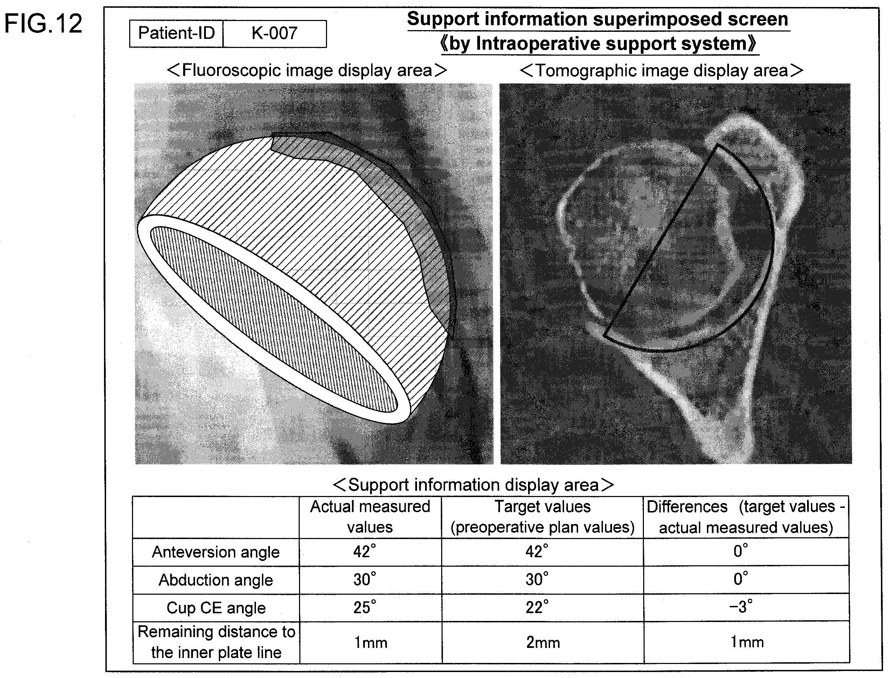

FIG. 12 is an explanatory diagram illustrating a display screen for support information (support information superimposed screen). As a result of the various processes described above, the processing unit 2 of the information processing apparatus 1 generates screen data that configures the support information superimposed screen, and outputs the screen data to the display device 41. The support information superimposed screen includes a fluoroscopic image display area, a tomographic image display area, and a support information display area.

In the fluoroscopic image display area, a fluoroscopic image (X-ray image) obtained by imaging the pelvis of the patient K in real time during surgery while the pelvis is being reamed by the bone reamer 7 is displayed, and the reach region is displayed so as to be superimposed on the fluoroscopic image. In the tomographic image display area, a tomographic image (CT image) including the current position of the bone reamer 7 identified in the X-ray image is displayed, and the outer edge diagram (semicircular figure object) of the cup 81 when the cup 81 is assumed to be placed with the pelvis reamed by the bone reamer 7 is displayed so as to be superimposed on the tomographic image. The reach region may also be displayed so as to be superimposed on the tomographic image.

In the support information display area, the anteversion angle, abduction angle, cup CE angle, and the remaining distance to the inner plate line when the cup 81 is assumed to be placed with the pelvis reamed by the bone reamer 7 are displayed in a list format. For the anteversion angle, abduction angle, cup CE angle, and the remaining distance to the inner plate line of the cup 81, the actual measured values calculated based on the current position and inclination of the bone reamer 7 identified by shape recognition processing in the fluoroscopic image, the values (target values) determined in the preoperative plan, and the differences between the actual measured values and the target values are displayed in a list format.

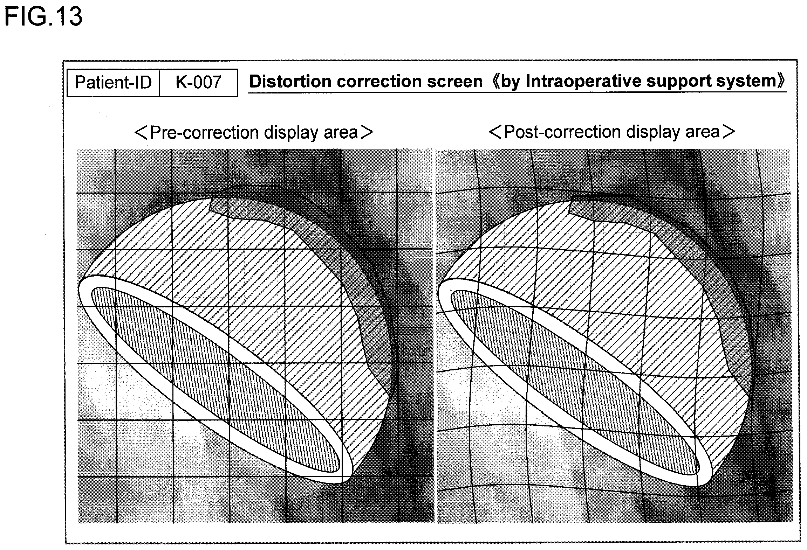

FIG. 13 is an explanatory diagram illustrating a display screen (distortion correction screen) for support information. As a result of the various processes described above, the processing unit 2 of the information processing apparatus 1 generates screen data that configures a distortion correction screen, and outputs the screen data to the display device 41. The distortion correction screen includes a pre-correction display area and a post-correction display area. Thus, since the distortion correction screen displays the fluoroscopic image before distortion correction (with support information superimposed) and the fluoroscopic image after distortion correction (with support information superimposed) in such a manner that these are compared with each other, it is possible to present doctors and others with information showing the before-and-after distortion correction with respect to the reach region (strike zone). Therefore, it is possible to provide useful information when the doctor performs treatment.

The distortion correction screen may be output as a screen separate from the support information superimposed screen described above, or may be included in the support information superimposed screen. When the distortion correction screen is output as a screen separate from the support information superimposed screen, the display device 41 that displays the distortion correction screen and the display device 41 that displays the support information superimposed screen may be separate display devices 41. In this case, two display devices 41 including the display device 41 that displays a distortion correction screen and the display device 41 that displays a support information superimposed screen are connected to the information processing apparatus 1.

In the pre-distortion correction display area, an image displayed in the fluoroscopic image display area on the support information superimposed screen described above (a fluoroscopic image on which the reach region is superimposed) is displayed. In addition, in the image displayed in the pre-correction display area (pre-correction image), grid-like auxiliary lines formed by a plurality of curves used in performing distortion correction are displayed.

In the post-correction display area, an image on which distortion correction has been performed (a fluoroscopic image on which the reach region is superimposed) is displayed. In addition, in the image displayed in the post-correction display area (post-correction image), the auxiliary lines used in performing distortion correction processing are displayed in a form that has been transformed into straight lines according to the correction. As a result, the reach region (strike zone) is also displayed with its shape (region on the pelvis) changed according to the distortion correction.

When performing distortion correction, the processing unit 2 of the information processing apparatus 1 may, for example, perform distortion correction processing by placing a template including straight lines below the fluoroscopic image (pre-correction image). In this case, the processing unit 2 of the information processing apparatus 1 is a device that converts X-rays that have passed through the human body into a digital image, and may be a device that uses a flat panel with a distortion correction function.

Alternatively, the processing unit 2 of the information processing apparatus 1 may perform distortion correction processing on the fluoroscopic image (pre-correction image) according to distortion aberration determined based on the X-ray imaging characteristics of the X-ray apparatus 62 or the like. The X-ray apparatus 62 that captures fluoroscopic images (X-ray images) has various characteristics depending on its model or type, and the distortion aberration corresponding to the amount of distortion in the captured fluoroscopic image (X-ray image) can also be identified. The storage unit 3 of the information processing apparatus 1 stores parameters such as distortion aberration corresponding to each of various X-ray apparatuses 62, and the processing unit 2 of the information processing apparatus 1 may perform distortion correction using the parameters such as distortion aberration corresponding to the X-ray apparatus 62 or the like. In this case, it is not necessary to use a flat panel, which is a dedicated device that performs distortion correction through hardware processing, and it is possible to display the distortion-corrected fluoroscopic image using a relatively inexpensive display (a display device that does not have a distortion correction function through hardware processing).

According to the present embodiment, the processing unit 2 of the information processing apparatus 1 acquires a fluoroscopic image (X-ray image) of the patient K undergoing a procedure related to the artificial hip joint 8. The fluoroscopic image is, for example, an X-ray image. As a procedure related to the artificial hip joint 8, when forming the cavity (installation space) of the bone serving as a receptacle in the pelvis for placing the cup 81 of the artificial hip joint 8 using the bone reamer 7 (when reaming the pelvis), fluoroscopic images (X-ray images) are captured in real time, and the processing unit 2 of the information processing apparatus 1 sequentially acquires the fluoroscopic images (X-ray images) captured in real time. The processing unit 2 of the information processing apparatus 1 recognizes (derives), in the pelvis of the patient K in the acquired fluoroscopic image, an inner plate line indicating the maximum reaming point when reaming the pelvis to place the cup 81 included in the artificial hip joint 8 and a target-to-reach line (a line that should be exceeded) indicating the minimum reaming point when reaming the pelvis to place the cup 81. In addition, the processing unit 2 of the information processing apparatus 1 derives the cup CE angle when the cup 81 is assumed to be placed with the pelvis reamed by the bone reamer 7 (in real time). The processing unit 2 of the information processing apparatus 1 displays the recognized (derived) inner plate line and target-to-reach line so as to be superimposed on the fluoroscopic image, and also displays the derived cup CE angle so as to be added to the fluoroscopic image by using, for example, a sub-screen. Therefore, it is possible to provide useful support information to the doctor performing a procedure related to the artificial hip joint 8 on the patient K. The processing unit 2 of the information processing apparatus 1 recognizes the shape of the outer edge of the bone reamer 7 (the outer edge of the hemispherical tip portion that performs reaming) included in the fluoroscopic image (X-ray image) by using an object detection system model such as YOLO or edge detection, for example. The processing unit 2 of the information processing apparatus 1 regards the outer edge of the bone reamer 7 as the outer edge of the cup 81 and derives the cup CE angle when the cup 81 is assumed to be placed with the pelvis reamed by the bone reamer 7. The processing unit 2 of the information processing apparatus 1 derives, as the cup CE angle, an angle formed by a line, which connects the center of rotation (COR) of the cup 81 assumed to be placed with the pelvis reamed by the bone reamer 7 to the intersection point between the outer edge of the cup 81 and the white line (Acetabular sourcil) of the pelvic weight-bearing surface, and the vertical line (Y-axis) with respect to the pelvic reference line, which connects the lower ends of the left and right teardrops in the pelvis. The processing unit 2 of the information processing apparatus 1 recognizes the shape of the lower ends of the left and right teardrops included in the fluoroscopic image (X-ray image) by using, for example, an object detection model such as YOLO or edge detection processing, and identifies the pelvic reference line that passes through these lower ends of the left and right teardrops. The processing unit 2 of the information processing apparatus 1 identifies a vertical line that is perpendicular to the identified pelvic reference line and passes through the center (COR) of the cup 81 that is assumed to be placed with the pelvis reamed by the bone reamer 7. The processing unit 2 of the information processing apparatus 1 derives the cup CE angle by calculating the angle formed by the vertical line passing through the center of rotation (COR) of the cup 81 and a line connecting the center of rotation of the cup 81 to the intersection point between the outer edge of the cup 81 and the white line (Acetabular sourcil) of the pelvic weight-bearing surface. The processing unit 2 of the information processing apparatus 1 acquires fluoroscopic images captured in real time, and derives the cup CE angle based on the outer edge of the bone reamer 7 included in the acquired fluoroscopic images. Therefore, it is possible to track the current position of the bone reamer 7, that is, the reaming state by the bone reamer 7, and provide the doctor with the cup CE angle when the cup 81 is placed at the current time. That is, it is possible to configure the intraoperative support system S that is capable of performing real-time intraoperative evaluation of the placement of the cup 81 and the coverage rate.

According to the present embodiment, one or more tomographic images obtained by imaging the pelvis of the patient K are, for example, CT images or MRI images, and are captured before the pelvis of the patient K is reamed by the bone reamer 7, and the processing unit 2 of the information processing apparatus 1 acquires the plurality of tomographic images and stores these in the storage unit 3. The storage unit 3 of the information processing apparatus 1 stores the actual file of the learning model 101 (inner plate line model), which has been trained to output the positional information (landmarks) of the inner plate when a tomographic image is input. The processing unit 2 of the information processing apparatus 1 inputs the acquired tomographic images (CT images and the like) to the learning model 101 (inner plate line model), and then the learning model 101 (inner plate line model) outputs the positional information of the inner plate so as to be superimposed on the tomographic image. Each piece of positional information of the inner plate may be output as a point (landmark), and a plurality of points (landmarks) may be output on the tomographic image. By forming (connecting) a line connecting the plurality of points (landmarks), the inner plate line may be identified. Thus, by using the learning model 101 (inner plate line model) that has been trained to output inner plate positional information (landmarks) when a tomographic image is input, it is possible to efficiently identify (derive) the inner plate line indicating the maximum reaming point when reaming the pelvis to place the cup 81 included in the artificial hip joint 8. The processing unit 2 of the information processing apparatus 1 performs alignment between a tomographic image (for example, a CT image) and a fluoroscopic image (for example, an X-ray image) based on the same internal body part (for example, a teardrop) identified using shape recognition or the like, and accordingly, can define these two images (tomographic image and fluoroscopic image) in the same internal body coordinate system and perform various arithmetic processes such as superimposing the images. The processing unit 2 of the information processing apparatus 1 displays (outputs) the inner plate line derived using a tomographic image (CT image and the like) so as to be superimposed on the fluoroscopic image (X-ray image) so that the same position (internal body coordinate value) as the inner plate line shown in the tomographic image is obtained. In the fluoroscopic image (X-ray image) displayed in real time during surgery, the bone reamer 7 at the current time is shown and the inner plate line is displayed so as to be superimposed on the fluoroscopic image (X-ray image). Therefore, it is possible to present doctors and others operating the bone reamer 7 with the inner plate line (reaming limit line) connected at the maximum reaming point when reaming the pelvis, that is, the limit point that should not be exceeded.

It should be considered that the embodiments disclosed this time are examples in all points and not restrictive. The scope of the present invention is defined by the claims rather than the meanings set forth above, and is intended to include all modifications within the scope and meaning equivalent to the claims.

A plurality of claims described in the claims can be combined with each other regardless of the citation form. In the claims, a multiple dependent claim that depends on a plurality of claims may be included. A multiple dependent claim that depends on a multiple dependent claim may be included. Even If a multiple dependent claim that depends on a multiple dependent claim is not included, this does not limit the inclusion of a multiple dependent claim that depends on a multiple dependent claim.

### Reference Signs List

- S: intraoperative support system
- K: patient
- 1: information processing apparatus
- 2: processing unit
- 3: storage unit
- M: recording medium
- P: program (program product)
- 4: input/output I/F
- 41: display device
- 5: communication unit
- 101: learning model (inner plate line model)
- 61: CT apparatus (tomographic image capturing apparatus)
- 62: X-ray apparatus (fluoroscopic image capturing apparatus)
- 7: bone reamer
- 8: artificial hip joint
- 81: cup

## Claims

1. An information processing method causing a computer to execute processing of:
acquiring a fluoroscopic image of a patient undergoing a procedure related to an artificial hip joint;
recognizing an inner plate line in a pelvis of the patient in the acquired fluoroscopic image, the inner plate line indicating a maximum reaming point when reaming the pelvis to place a cup included in the artificial hip joint;
recognizing a target-to-reach line in the pelvis of the patient, the target-to-reach line indicating a minimum reaming point when reaming the pelvis to place the cup;
deriving a cup CE angle when the cup is assumed to be placed with the pelvis of the patient reamed by a bone reamer used in the procedure related to the artificial hip joint;
displaying the recognized inner plate line and the recognized target-to-reach line so as to be superimposed on the fluoroscopic image; and
displaying the derived cup CE angle so as to be added to the fluoroscopic image.

2. The information processing method according to claim 1,
wherein a tomographic image obtained by imaging the pelvis of the patient is acquired,
positional information of the inner plate is derived by inputting the acquired tomographic image to a learning model that has been trained to output positional information of an inner plate when a tomographic image is input, and
the inner plate line in the fluoroscopic image is recognized based on the output group of positional information of the plurality of inner plates.

3. The information processing method according to claim 1,
wherein an upward limit line for a femoral head center on an affected side where the procedure related to the artificial hip joint is to be performed is derived based on a femoral head center on a healthy side where the procedure related to the artificial hip joint is not to be performed,
a reach region is derived based on the derived upward limit line, the derived inner plate line, and the derived target-to-reach line, and
the derived reach region is displayed so as to be superimposed on the fluoroscopic image.

4. The information processing method according to claim 1,
wherein the target-to-reach line is derived based on a target CE angle set in advance to stabilize the cup to be placed, an intersection point between an outer edge of the cup and an inner edge of a weight-bearing surface of the pelvis of the patient, which serves as a reference when ensuring the target CE angle, and a radius of the cup.

5. The information processing method according to claim 1,
wherein the derived cup CE angle is output so as to be compared with a target CE angle set in advance as a target value,
an actual measured distance between an outer edge of the cup and the inner plate line when the cup is assumed to be placed with the pelvis of the patient reamed by the bone reamer is derived,
a target distance between the outer edge of the cup and the inner plate line, which is set in advance as a target value, is derived, and
the derived actual measured distance and the derived target distance are output so as to be compared with each other.

6. The information processing method according to claim 1,
wherein an anteversion angle and an abduction angle of the cup when the cup is assumed to be placed with the pelvis of the patient reamed by the bone reamer is derived, and
the derived anteversion angle and abduction angle are output.

7. The information processing method according to claim 5,
wherein a target anteversion angle and a target abduction angle as targets are derived according to the target CE angle, and
the derived target anteversion angle and the derived target abduction angle are output in combination.

8. The information processing method according to claim 7,
wherein the derived anteversion angle and abduction angle of the cup and the target anteversion angle and the target abduction angle are output so as to be compared with each other.

9. The information processing method according to claim 2,
wherein a current position of the bone reamer in the pelvis of the patient is acquired based on the acquired fluoroscopic image,
a tomographic image including the current position of the bone reamer is extracted from among the plurality of tomographic images obtained by imaging the pelvis of the patient, and
an outer edge diagram of the cup when the cup is assumed to be placed with the pelvis of the patient reamed by the bone reamer is derived is output so as to be superimposed on the extracted tomographic image.

10. A program that causes a computer to execute processing of:
acquiring a fluoroscopic image of a patient undergoing a procedure related to an artificial hip joint;
recognizing an inner plate line in a pelvis of the patient in the acquired fluoroscopic image, the inner plate line indicating a maximum reaming point when reaming the pelvis to place a cup included in the artificial hip joint;
recognizing a target-to-reach line in the pelvis of the patient, the target-to-reach line indicating a minimum reaming point when reaming the pelvis to place the cup;
deriving a cup CE angle when the cup is assumed to be placed with the pelvis of the patient reamed by a bone reamer used in the procedure related to the artificial hip joint;
displaying the recognized inner plate line and the recognized target-to-reach line so as to be superimposed on the fluoroscopic image; and
displaying the derived cup CE angle so as to be added to the fluoroscopic image.

11. An information processing apparatus, comprising:
an acquisition unit that acquires a fluoroscopic image of a patient undergoing a procedure related to an artificial hip joint;
an inner plate line recognition unit that recognizes an inner plate line in a pelvis of the patient in the acquired fluoroscopic image, the inner plate line indicating a maximum reaming point when reaming the pelvis to place a cup included in the artificial hip joint;
a target-to-reach line recognition unit that recognizes a target-to-reach line in the pelvis of the patient, the target-to-reach line indicating a minimum reaming point when reaming the pelvis to place the cup;
a derivation unit that derives a cup CE angle when the cup is assumed to be placed with the pelvis of the patient reamed by a bone reamer used in the procedure related to the artificial hip joint; and
a display unit that displays the recognized inner plate line and the recognized target-to-reach line so as to be superimposed on the fluoroscopic image and displays the derived cup CE angle so as to be added to the fluoroscopic image.
